# EUROPÄISCHE PATENTSCHRIFT

(11) **EP 1 284 675 B1**
(45) Veröffentlichungstag und Bekanntmachung des Hinweises auf die Patenterteilung: **03.01.2007**
(21) Anmeldenummer: 01911685.4
(22) Anmeldetag: 22.02.2001
(51) Int. Cl.: A61C 8/00, A61B 17/16

(54) **VORRICHTUNG MIT EINEM KNOCHENFRÄSER**
DEVICE PROVIDED WITH A BONE CUTTER
DISPOSITIF POURVU D'UNE FRAISE A OS

(30) Priorität: 25.02.2000 AT 3032000
(43) Veröffentlichungstag der Anmeldung: 26.02.2003
(73) Patentinhaber: DeguDent GmbH, 63457 Hanau-Wolfgang (DE)
(72) Erfinder: JESCH, Wolfgang, A-3001 Mauerbach (AT)
(74) Vertreter: Stoffregen, Hans-Herbert
(86) Internationale Anmeldenummer: PCT/EP2001/002015
(87) Internationale Veröffentlichungsnummer: WO 2001/062179

(56) Entgegenhaltungen:
- WO-A-98/48707
- DE-A- 19 801 181
- FR-A- 2 749 158
- PATENT ABSTRACTS OF JAPAN vol. 018, no. 086 (C-1165), 14. Februar 1994 (1994-02-14) -& JP 05 293124 A (TDK CORP), 9. November 1993 (1993-11-09)

## Beschreibung

Die Erfindung bezieht sich auf eine Vorrichtung mit einem Knochenfräser zum Ansetzen an medizinische, insbesondere zahnmedizinische Handstücke, beispielsweise Winkelstücke, wobei der Fräser mit seinem Fräskopf in einer Buchse in Richtung Drehachse des Knochenfräsers verschiebbar gelagert ist und an der Stirnfläche der Buchse eine eine Stanze bildende kreisbogenförmige Messerkante vorgesehen ist, und wobei der Fräskopf aus der Ebene der Stirnfläche der Buchse ausschiebbar ist.

Zum Setzen des Zahnimplantats ist es bisher üblich, die Schleimhaut vom Kieferknochen operativ abzulösen und das Periost zu spalten. Danach wird dann das Implantat entsprechend den Herstelleranweisungen in den Knochen eingesetzt. Die Operationswunde wird anschließend durch Setzen von mehreren Nähten verschlossen. Eine solche Vorgehensweise hat den Nachteil, dass die Schleimhaut, also die Gingiva, traumatisch durchdrungen wird, wobei nach Beendigung der Arbeit der Patient längere Zeit auch an Wundschmerz leiden kann.

Der WO 98/48707 ist eine gattungsgemäße Vorrichtung zu entnehmen. Dabei wird zum Entfernen von Gewebe eine endseitig eine Schneidkante aufweisende Hülse in das Gewebe eingepresst. Zur Erleichterung kann die Hülse hin-und herbewegt werden.

Die DE 098 01 181 A1 bezieht sich auf eine Vorrichtung zur Entnahme von Knochenteilen und umfasst einen Zentralbohrer, der von einem Kembohrer umgeben ist. Kernbohrer und Zentralbohrer sind kraftschlüssig verbunden, wobei die Schneidfläche des Kernbohrers zu der des Zentralbohrers zurückversetzt verläuft.

Der Erfindung liegt die Aufgabe zu Grunde, ein möglichst atraumatisches Durchdringen der Gingiva und des Periosts zu erreichen, wobei zusätzlich ein kreisrundes, kegelförmiges Plateau im Bereich des Alveolarkammes des Oberkiefers bzw. Unterkiefers geschaffen werden soll.

Erfindungsgemäß wird die Aufgabe dadurch gelöst, dass die Buchse drehschlüssig mit dem Fräser gekuppelt ist. Dadurch wird erreicht, dass vor dem Wirksamwerden des Fräskopfes die rotierende Stanze das atraumatische Heraustrennen der Gingiva und des Periosts durchführt, wonach durch den Fräskopf einerseits die herausgeschnittenen Gewebeteile zerkleinert werden und andererseits nach entsprechendem Absenken des Fräskopfes auf den Alveolarkamm die entsprechende Ansatzfläche erzielt wird.

Vorteilhafterweise kann der Fräser mit seinem Schaft eine Glocke durchsetzen, die Glocke unverschiebbar mit dem Fräserschaft verbunden sein und die die rotierende Stanze bildende Buchse gleitbar gegen den Druck einer Feder in der Glocke geführt sein, wobei die Feder an der Glocke und an der Buchse abgestützt ist. Dadurch wird einerseits eine zuverlässige Führung der Buchse an der Außenseite des Fräsers erreicht und außerdem auch ein Aufnahmeteil für die Feder gebildet. Für ein sicheres Mitnehmen der Buchse mit der Rotation des Fräsers kann der Schaft des Fräsers einen zumindest sich über einen Teil seiner Länge erstreckenden Abschnitt mit unrundem (von der Kreisform abweichenden) Querschnitt aufweisen, der formschlüssig eine korrespondierend unrund ausgebildete Öffnung in der die rotierende Stanze bildenden Buchse durchsetzt. In besonders vorteilhafter Weise kann der Fräskopf stirnseitige Schneidkanten aufweisen, die gegen die Drehachse des Fräsers unter einem von 90° verschiedenen, bevorzugt 60° betragenden Winkel geneigt sind, wodurch erreicht wird, daß eine gleichförmige Ansatzfläche für das Einsetzen des Implantats gegeben ist. Dabei können die Schneidkanten des Fräskopfes an der Stirnkante eines Schneidplättchens ausgebildet sein, das eine Mittenachse besitzt, die mit der Drehachse des Fräsers zusammenfällt, wobei die Schneidkanten symmetrisch zur Mittenachse des Schneidplättchens angeordnet sind und miteinander einen Winkel (□) von bevorzugt 120° einschließen, wobei der Scheitel des Winkels in der Drehachse des Fräsers liegt, wodurch erreicht wird, daß vom Alveolarkamm abgelöstes Material nicht in die Alveolaröffnung hineingepreßt wird, sondern im Zwischenraum zwischen Innenwandung der Buchse und den Rauten des Schneidplättchens verbleiben kann.

Um zu verhindern, daß durch die Federkraft die Buchse selbsttätig über die Spitze des Fräserkopfes abfallen kann, kann die Buchse, z. B. durch Anschläge, gegen ein in Richtung zum Fräserkopf gerichtetes Abgleiten vom Fräser gesichert sein. Schliesslich können in besonders einfacher Weise als Anschläge am Fräserkopf an seinem der Schneidkante abgewandten Ende eine Schulter vorgesehen sein, an welcher den unrunden Querschnitt verursachende Vorsprünge der Buchsenbohrung zur Anlage gelangen, was zudem ein einfaches Zusammenbauen des Gerätes ermöglicht.

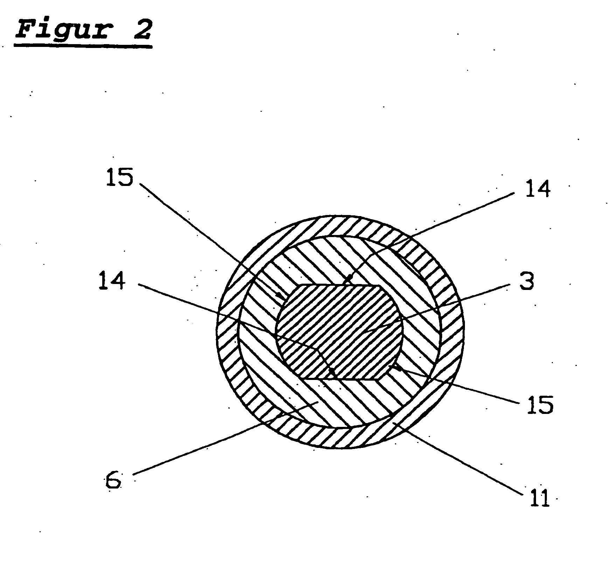

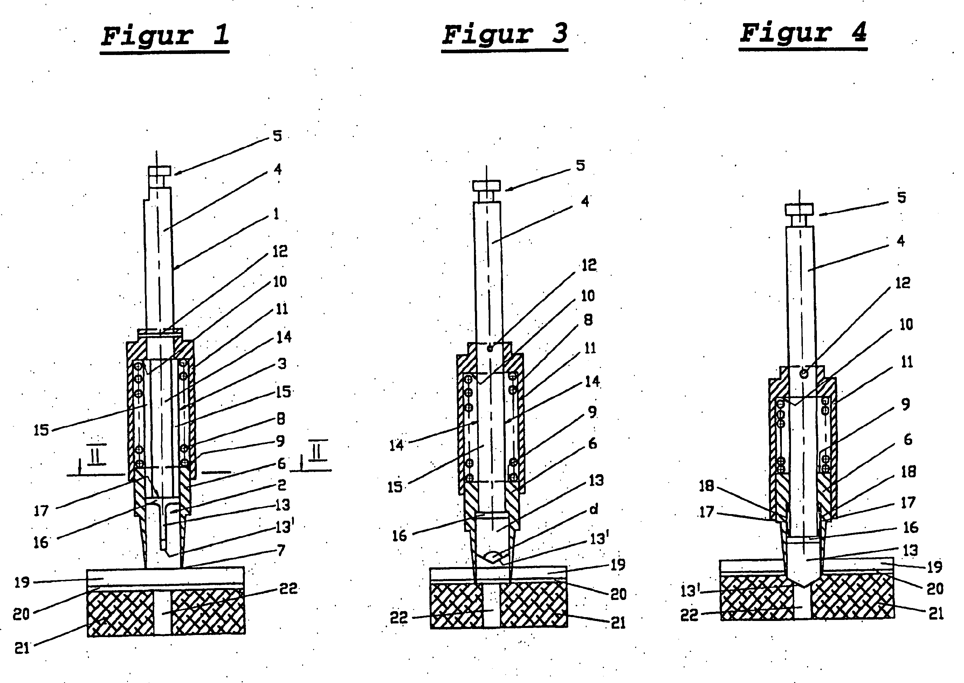

In der Zeichnung ist ein Ausführungsbeispiel des Erfindungsgegenstandes in wesentlich vergrößertem Maßstab dargestellt. Fig. 1 zeigt einen Vertikalschnitt durch die Vorrichtung. Fig. 2 ist ein Schnitt nach Linie II - II der Fig. 1. Fig. 3 gibt einen der Fig.1 analogen Schnitt wieder, jedoch bei um 90° um die Längsachse verdrehter Schnittebene, und zwar bei bereits durchdrungene Gingiva, wobei die vordere Schneidkante am Alviolarkamm aufsitzt. Fig. 4 ist eine der Fig. 3 analoge Schnittdarstellung, jedoch bei vollkommen abgesenktem Fräskopf.

Mit 1 ist ein Fräser bezeichnet, der aus einem Fräskopf 2, einem Schaftteil mit unrundem Querschnitt 3 und einem an diesen anschließenden Schaftteil mit rundem Querschnitt 4 besteht, wobei am freien Ende des nach oben herausragenden Schaftteiles mit rundem Querschnitt 4 die Ansatzkupplung 5 für das Ansetzen des Handstückes bzw. Winkelstückes vorgesehen ist.

Der Fräser 1 wird im unteren Bereich, und zwar im Bereich seines Fräskopfes 2 und teilweise auch im Bereich des Schaftteiles mit unrundem Querschnitt 3, von einer Buchse 6 umgeben, deren Innenquerschnitt dem Außenquerschnitt des Schaftteiles 3 mit unrundem Querschnitt entspricht. Diese Buchse weist an ihrem freien Ende eine Stirnfläche 7 auf, welche als kreisbogenförmige Messerkante ausgebildet ist und damit die Buchse eine rotierende Stanze bildet. An dem der Stirnfläche 7 der Buchse abgewandten Bereich derselben ist eine den Schaftbereich 3 umgebende Feder 8 vorgesehen, deren eines Ende sich an der hinteren Stirnfläche 9 der Buchse 6 und deren anderes Ende sich an einer inneren Stirnfläche 10 einer die Buchse außen umgebenden zylindrischen Glocke 11 abstützt. Diese zylindrische Glocke 11 ist mit dem runden Schaftbereich 4 drehschlüssig und in Axialrichtung unverschieblich verbunden, wobei im vorliegenden Ausführungsbeispiel ein Federstift 12 zur Festlegung vorgesehen ist.

Durch diese Ausbildung ist vorgesehen, daß die Buchse 6 in das Innere der Glocke 11 zurückgeschoben werden kann, und zwar entgegen der Kraft der Feder 8. Durch dieses Zurückschieben wird der Fräserkopf 2 freigesetzt, welcher vorliegend als Schneidplatte 13 ausgebildet ist, deren vordere Kante 13' abgewinkelt ist, und zwar derart, daß die beiden Schneidkanten zueinander einen Winkel □ von etwa 120° aufweisen.

Der Schaftbereich 3 mit unrundem Querschnitt besteht dabei aus einem im Wesentlichen zylindrischen Teil, der an zwei parallel gegenüberliegenden Flanken abgeflacht ist. Die Abflachungen sind in Fig. 2 mit 14 bezeichnet. Diese beiden Abflachungen sind demgemäß dann mit Zylindermantelflächen 15 untereinander verbunden. Diese Zylindermantelflächen 15 dienen als Führungsflächen für die Feder 8.

Der Fräskopf 2 weist an seinem der Schneidkante 13 abgwandten Bereich am Übergang zum Schaft 3 einen Bund 16 auf, der im Bereich der Abflachungen 14, der Schneidplatte abgewandt, Schultern 17 besitzt, die als Anschlag für an der Innenseite der Buchse 6 vorgesehene Vorsprünge 18 dienen Diese Anschläge 17,18 sind deshalb vorgesehen, um durch die Feder 8 bewirktes Herausgleiten der Buchse 6 aus der Glocke 11 zu verhindern.

Mit 19 ist schematisch die Gingiva, mit 20 das Peroist mit 21 der Alveolarkamm und mit 22 eine Alveole bezeichnet.

Wie eingangs angeführt, ist der Erfindungsgegenstand in einem sehr stark vergrößerten Maßstab wiedergegeben. Die tatsächlichen Maße liegen hingegen im Millimeterbereich, und zwar ist der Durchmesser der Buchse 6 im Bereich der Schneidkante 7 etwa 3mm und die Gesamtlänge der Vorrichtung bei voll vorgeschobener Buchse 6, wie in Fig. 1 dargestellt, beträgt etwa 33,5mm. Es ist also erkennbar, daß es sich hier um eine Vorrichtung handelt, die sehr klein gebaut sein muß, da sonst Schwierigkeiten beim Ansetzen am Kiefer auftreten können, insbesondere wenn es sich um Implantate handelt, die im hinteren Bereich der Mundhöhle einzusetzen sind.

Bei Betrieb der Vorrichtung wird an das Ansatzstück 5 ein Winkelstück oder ein Handstück angesetzt, wonach dann nach Einschalten des Motors und Einrichten des Kühlwasserstrahls, auf das Schneidmesser 7 die Einrichtung auf die Gingiva oberhalb der zu bearbeitenden Alveole aufgesetzt wird. Durch die Rotation des Fräserschaftes wird auch die Buchse 6 aufgrund der unrunden Ausbildung des Schaftbereichs 3 und der damit deckungsgleichen Ausbildung des Innenquerschnitts der Buchse in Rotation versetzt, so dass bei leichtem Andrücken die Schneidkante 7 die Gingiva und das Periost durchtrennt, also in diese beiden Gewebsbereiche kongruente Löcher hineinstanzt, wobei dann die Schneidkante 7 der Buchse 6 auf dem Alveolarkamm 21 aufsitzt. Dieser Zustand ist in Figur 3 wiedergegeben. Bei weiterem Abwärtsdrücken des Schaftes wird die Buchse 6 in Bezug auf die Glocke 11 nach hinten geschoben und das im Buchsenraum befindliche Gewebsmaterial der Gingiva und des Periosts durch die Schneidkante des Fräserkopfes 2 zerkleinert. Dies geschieht solange, bis die Schneidplatte 13 mit ihren Vorderkanten 13' auf den Alveolarkamm auftrifft und das obere Ende der Alveole so anschrägt, daß der Alveolarkamm im Bereich der Alveole eine durchgehend umlaufende Kegelmantelfläche aufweist. An der Kegelmantelfläche kann dann der eigentliche Implantatbohrer angesetzt und zentriert werden.

Aufgrund des Herausstanzens des oberhalb der Alveole 22 liegenden Bereiches der Gingiva 19 und des Periosts 20 erfolgt eine atraumatische Entfernung dieser Gewebsbereiche, da bei den herkömmlichen Methoden nach dem Ablösen der Gingiva vom Kieferknochen und dem Spalten des Periosts die überflüssigen Gewebsteile abgeschnitten werden müssen, wobei für das Ablösen der Gingiva oder Spalten des Periosts ein größerer Bereich der Gingiva aufzutrennen ist, als dies bei dem Herausstanzen gemäß vorliegender Erfindung erforderlich ist.

## Patentansprüche

1. Vorrichtung mit einem Knochenfräser zum Ansetzen an medizinische, insbesondere zahnmedizinische Handstücke, beispielsweise Winkelstücke, wobei der Fräser (1) mit seinem Fräskopf (2) in einer Buchse (6) in Richtung Drehachse des Knochenfräsers verschiebbar gelagert ist und an der Stirnfläche (7) der Buchse (6) eine eine Stanze bildende kreisbogenförmige Messerkante vorgesehen ist, und wobei der Fräskopf (2) aus der Ebene der Stirnfläche (7) der Buchse (6) ausschiebbar ist.
**dadurch gekennzeichnet,**
**dass** die Buchse (6) drehschlüssig mit dem Fräser (1) gekuppelt ist.

2. Vorrichtung nach Anspruch 1,
**dadurch gekennzeichnet,**
**daß** der Fräser (1) mit seinem Schaft (3,4) eine Glocke (11) durchsetzt und daß die Glocke unverschiebbar mit dem Fräserschaft (4) verbunden ist und die die rotierende Stanze bildende Buchse (6) gleitbar gegen den Druck einer Feder (8) in der Glocke (11) geführt ist, wobei die Feder (8) an der Glocke (11) und an der Buchse (6) abgestützt ist.

3. Vorrichtung nach Anspruch 1 oder 2 ,
**dadurch gekennzeichnet,**
**daß** der Schaft (3,4) des Fräsers (1) einen zumindest sich über einen Teil seiner Länge erstreckenden Abschnitt (3) mit unrundem, von der Kreisform abweichenden, Querschnitt aufweist, der formschlüssig eine korrespondierend unrund ausgebildete Öffnung in der die rotierende Stanze bildenden Buchse (6) durchsetzt.

4. Vorrichtung nach einem der Ansprüche 1 bis 3,
**dadurch gekennzeichnet,**
**daß** der Fräskopf (2) stirnseitig Schneidkanten (13') aufweist, die gegen die Drehachse des Fräsers (2) unter einem von 90° verschiedenen, bevorzugt 60° betragenden Winkel geneigt sind.

5. Vorrichtung nach Anspruch 4
**dadurch gekennzeichnet,**
**daß** die Schneidkanten (13') des Fräskopfes (2) an der Stirnkante eines Schneidplättchens (13) ausgebildet sind, das eine Mittenachse besitzt, die mit der Drehachse des Fräsers (1) zusammenfällt, wobei die Schneidkanten (13') symmetrisch zur Mittenachse des Schneidplättchens (13) angeordnet sind und miteinander einen Winkel (□) von bevorzugt 120° einschließen, wobei der Scheitel des Winkels in der Drehachse des Fräsers liegt.

6. Vorrichtung nach einem der Ansprüche 1 bis 5,
**dadurch gekennzeichnet,**
**dass** die Buchse (6) durch Anschläge gegen ein in Richtung zum Fräskopf (2) gerichtetes Abgleiten vom Fräser (1) gesichert ist.

7. Vorrichtung nach Anspruch 6,
**dadurch gekennzeichnet,**
**daß** als Anschläge am Fräserkopf (2) an seinem der Schneidkante (7) abgewandten Ende eine Schulter (17) vorgesehen ist, an welcher den unrunden Querschnitt verursachende Vorsprünge (18) der Buchsenbohrung zur Anlage gelangen.

## Claims

1. Device with a bone milling cutter for application on medical, especially dental handpieces, for example angle pieces, where the milling cutter (1) with its milling head (2) is mounted so as to be displaceable in a bushing (6) towards axis of ration of the bone milling cutter and a circular knife edge forming a punch is provided on the face (7) of the bushing (6), and where the milling head (2) can be pushed out of the plane of the face (7) of the bushing (6),
**characterized in**
**that** the bushing (6) is coupled rotation-locking with the bone milling cutter (1).

2. Device according to claim 1,
**characterized in**
**that** the milling cutter (1) with its shaft (3, 4) penetrates a bell (11), and that the bell is fixedly connected to the milling cutter shaft (4), and the bushing (6) forming the rotating punch is guided so as to slide against the pressure of a spring (8) in the bell (11), where the spring (8) is braced on the bell (11) and on the bushing (6).

3. Device according to claim 1 or 2,
**characterized in**
**that** the shaft (3, 4) of the milling cutter (1) has a portion extending at least over a part of its length and having a non-circular (deviating from circular) cross section, which penetrates form-fittingly through a correspondingly non-circularly configured opening in the bushing (6), which forms the rotating punch.

4. Device according to one of the claims 1 to 3,
**characterized in**
**that** the milling head (2) has cutting edges (13') on its front face which are inclined against the axis of rotation of the milling cutter (2) at an angle different from 90° preferably amounting to 60°.

5. Device according to claim 4,
**characterized in**
**that** the cutting edges (13') of the milling head (2) are formed on the front face edge of a cutting platelet (13) that has a central axis which coincides with the axis of rotation of the milling cutter (1), where the cutting edges (13') are arranged symmetrically with respect to the central axis of the cutting platelet (13) and together enclose an angle (□) of preferably 120°, where the crest of the angle lies in the axis of rotation of the milling cutter.

6. Device according to one of the claims 1 to 5,
**characterized in**
**that** the bushing (6) is secured by stops against a slipping of the milling cutter (1) oriented in the direction toward the milling cutter head (2).

7. Device according to claim 6,
**characterized in**
**that** a shoulder (17) is provided as stops on the milling head (2) on its end facing away from the cutting edge (7) on which the projections (18) of the bushing borehole causing the non-circular section get into position.

## Revendications

1. Dispositif comportant une fraise à os pouvant être rapportée sur des porte-outils, en particulier des porte-outils de la technique dentaire, par exemple des raccords angulaires, la fraise (1) étant logée avec sa tête de fraisage (2) dans une douille (6) et déplaçable dans la direction de l'axe de rotation de la fraise à os, avec sur la face frontale (7) de la douille (6) une arête coupante en arc de cercle formant une estampe, et la tête de fraise (2) pouvant coulisser hors du plan de la face frontale (7) de la douille (6),
**caractérisé en ce que**
la douille (6) est couplée à la fraise (1) en liaison de rotation.

2. Dispositif selon la revendication 1,
**caractérisé en ce que**
la fraise (1) traverse avec sa tige (3, 4) une cloche (11), et la cloche est reliée à la tige de fraise (4) de façon fixe et la douille (6) formant l'estampe rotative est guidée de façon coulissante à l'encontre la pression d'un ressort (8) dans la cloche (11), le ressort (8) prenant appui contre la cloche (11) et la douille (6).

3. Dispositif selon la revendication 1 ou 2,
**caractérisé en ce que**
la tige (3, 4) de la fraise (1) présente, au moins sur une partie de sa longueur, un segment (3) d'une section transversale non ronde différant de la forme circulaire, qui traverse avec complémentarité de forme une ouverture non ronde complémentaire dans la douille (6) formant l'estampe rotative.

4. Dispositif selon l'une quelconque des revendications 1 à 3,
**caractérisé en ce que**
sur la face frontale la tête de fraise (2) présente des arêtes tranchantes (13') inclinées par rapport à l'axe de rotation de la fraise (2) sous un angle différent de 90°, de préférence un angle de 60°.

5. Dispositif selon la revendication 4,
**caractérisé en ce que**
les arêtes tranchantes (13') de la tête de fraisage (2) sont formées sur l'arête frontale d'une plaquette tranchante (13) qui présente un axe médian coïncidant avec l'axe de rotation de la fraise (1), les arêtes tranchantes (13') étant symétriques par rapport à l'axe médian de la plaquette tranchante (13) et enfermant de préférence un angle (□) de 120°, le sommet de l'angle se situant dans l'axe de rotation de la fraise.

6. Dispositif selon l'une quelconque des revendications 1 à 5,
**caractérisé en ce que**
la douille (6) est bloquée par des butées contre un glissement de la fraise (1) en direction de la tête de fraisage (2)

7. Dispositif selon la revendication 6,
**caractérisé en ce que**
comme butées un épaulement (17) est prévu sur la tête de fraisage (2) à son extrémité opposée à l'arête tranchante (7), contre lequel s'appliquent des saillies (18) de l'alésage de douille qui engendrent la section transversale non ronde.
